# EUROPEAN PATENT SPECIFICATION

(11) **EP 4 469 139 B1**
(45) Date of publication and mention of the grant of the patent: **11.03.2026**
(21) Application number: 23719490.7
(22) Date of filing: 31.03.2023
(51) Int. Cl.: A61N 1/05, A61N 1/36, A61N 1/378

(54) **DELIVERING TUMOR TREATING FIELDS (TTFIELDS) USING IMPLANTED SHEETS OF GRAPHITE**
ABGABE VON TUMORBEHANDLUNGSFELDERN (TTFIELDS) UNTER VERWENDUNG IMPLANTIERTER GRAPHITFOLIEN
ADMINISTRATION DE CHAMPS DE TRAITEMENT DE TUMEUR (TTFIELDS) À L'AIDE DE FEUILLES DE GRAPHITE IMPLANTÉES

(30) Priority: 31.03.2022 US 202263325650 P
(43) Date of publication of application: 04.12.2024
(73) Proprietor: Novocure GmbH, 6340 Baar (CH)
(72) Inventor: WASSERMAN, Yoram, 31905 Haifa (IL); OBUCHOVSKY, Stas, 31905 Haifa (IL); KUPLENNIK, Nataliya, Kanata, Ontario K2W1E7 (CA); SHAPIRO, David, 31905 Haifa (IL)
(74) Representative: Boden, Keith McMurray
(86) International application number: PCT/IB2023/053279
(87) International publication number: WO 2023/187755

(56) References cited:
- CN-A- 113 769 267
- CN-A- 113 926 079
- KR-A- 20200 016 102
- US-A1- 2006 276 858
- US-A1- 2019 224 474
- SEOK JOO KIM ET AL: "Stretchable and Transparent Biointerface Using Cell-Sheet-Graphene Hybrid for Electrophysiology and Therapy of Skeletal Muscle", ADVANCED FUNCTIONAL MATERIALS, WILEY - V C H VERLAG GMBH & CO. KGAA, DE, vol. 26, no. 19, 15 March 2016 (2016-03-15), pages 3207 - 3217, XP072411382, ISSN: 1616-301X, DOI: 10.1002/ADFM.201504578

## Description

### BACKGROUND

TTFields (Tumor Treating Fields) therapy is a proven approach for treating tumors using alternating electric fields at frequencies between 50 kHz - 1 MHz, such as from 100 kHz-500 kHz. In the prior art Optune^{®} system for delivering TTFields, the TTFields are delivered to patients via four transducer arrays placed on the patient's skin in close proximity to a tumor. The transducer arrays are arranged in two pairs. One of those pairs is positioned on the left and right sides of the target region (e.g., a glioblastoma); and the other one of those pairs is positioned on the front and back of the target region. Each transducer array is connected via a cable to an AC voltage generator. The AC voltage generator (a) sends an AC current through one pair of arrays during a first period of time; then (b) sends an AC current through the other pair of arrays during a second period of time; then repeats steps (a) and (b) for the duration of the treatment.

KR-A-20200016102 discloses a deep brain stimulating transparent electrode array which includes a transparent graphene neural electrode including a biocompatible dielectric substrate, a plurality of electrode sites which are disposed on one side of the substrate and include a graphene sheet, a plurality of electroconductive contacts which are disposed on the other side of the substrate, and an electroconductive interconnector which connects the electrode sites and the contacts.

CN-A-113926079 discloses an implantable electrode slice which is arranged in a target biological tissue area, and comprises an electric field electrode part and a detection part, where the electric field electrode part is configured to be electrically connected with an electric signal generation unit and forms a target electric field at least surrounding the target biological tissue area according to a received electric signal of the electric signal generation unit, and the detection part is configured to be electrically connected with a monitoring unit and detects an electric signal representing biological characteristics of the target biological tissue area.

### SUMMARY OF THE INVENTION

One aspect of this application is directed to an apparatus for applying an alternating electric field to a target region in a subject's body according to claim 1.

Embodiments of the invention are disclosed in the dependent claims.

### BRIEF DESCRIPTION OF THE DRAWINGS

FIG. 1A depicts two sheets of graphite that are implanted in a subject's body on opposite sides of a target region.
FIG. 1B depicts a variation of the FIG. 1A embodiment that includes an additional layer of material.
FIG. 2 depicts a plan view of an apparatus for applying an alternating electric field to the target region in a subject's body.
FIG. 3 is a side view of the FIG. 2 apparatus.
FIG. 4 depicts a variation of the FIG. 2-3 embodiment that includes two additional layers of material.
FIG. 5 depicts another variation of the FIG. 2-3 embodiment that includes additional layers of material.
FIGS. 6 and 7 are plan and side views of another apparatus for applying an alternating electric field to a target region in a subject's body.
FIG. 8 is a side view of another apparatus for applying an alternating electric field to a target region in a subject's body.
FIG. 9 is a side view of another apparatus for applying an alternating electric field to a target region in a subject's body.
FIG. 10 is a side view of another apparatus for applying an alternating electric field to a target region in a subject's body.
FIG. 11 depicts a variation of the FIG. 1A embodiment that utilizes a single multi-conductor port.

Various embodiments are described in detail below with reference to the accompanying drawings, wherein like reference numerals represent like elements.

### DESCRIPTION OF THE PREFERRED EMBODIMENTS

Instead of using transducer arrays positioned on the patient's skin to deliver TTFields, the embodiments described herein use electrodes that are implanted within a patient's body to deliver TTFields. Implanting the electrodes can provide a number of potential advantages. These potential advantages include (1) hiding of the arrays from people with whom the patient interacts; (2) improving patient comfort (by avoiding the skin irritation, sensations of heating, and/or limitations on motion that can result from arrays that are positioned on the patient's skin); (3) improving electrical contact between the electrodes and the patient's body; (4) eliminating the need for shaving regions on which the arrays are placed (as hair growth can interfere with the delivery of TTFields); (5) avoiding the risk that detachment of the electrodes will interrupt the delivery of TTFields; (6) significantly reducing the power required to deliver TTFields (e.g., by reducing the physical distance between the electrodes and the tumor and bypassing anatomical structures that have high resistivity, e.g., the skull); (7) significantly reducing the weight of the device that must be carried by the patient (e.g., by using smaller batteries to take advantage of the reduced power requirements); (8) avoiding the skin irritation that can occur when transducer arrays are positioned on the patient's skin; and (9) making it possible to deliver TTFields to anatomic structures that cannot be treated using transducer arrays positioned on the patient's skin (e.g., the spinal cord, which is surrounded by highly conductive cerebrospinal fluid that is in turn surrounded by the bony structure of the spine, both of which interfere with the penetration of TTFields into the spinal cord itself).

Improvements are sought over the prior art implantable medical devices for delivering alternating electric fields to a target region within a subject's body. Such devices have been bulky due to the number of electrodes required in an array and the size of the electrode elements in the array. Moreover, the operating currents have been low (typically below 1 A) because of the need to avoid hot spots that could damage the tissue in the body, and the low operating current has limited their efficacy. The present application addresses these and other important issues.

Preferably, each of the electrodes that is implanted within a patient's body is formed using a sheet of graphite. Using graphite sheets provides significant advantages with respect to other materials because graphite sheets spread the electrical current (which arrives from the AC signal generator) out in directions that are parallel to the front face of the sheet, and also spread the heat out in directions that are parallel to the front face of the sheet. These two advantages are significant when the graphite sheet is a sheet of high purity exfoliated mineral graphite. And these two advantages can be even more significant when the graphite sheet is a sheet of synthetic graphite, such as a sheet of pyrolytic graphite, or graphitized polymer film (e.g., graphitized polyimide). Because the current and heat in these embodiments are both spread out over a larger area of the front face of each electrode, hot spots are eliminated (or at least minimized). Accordingly, the surface area of the electrodes can be reduced without generating excessive heat and hot spots which would necessitate operating at a lower current; and/or the current can be increased (with respect to embodiments that do not include a graphite sheet). And this increase in current will advantageously increase the efficacy of the TTFields treatment. Moreover, graphite is biocompatible.

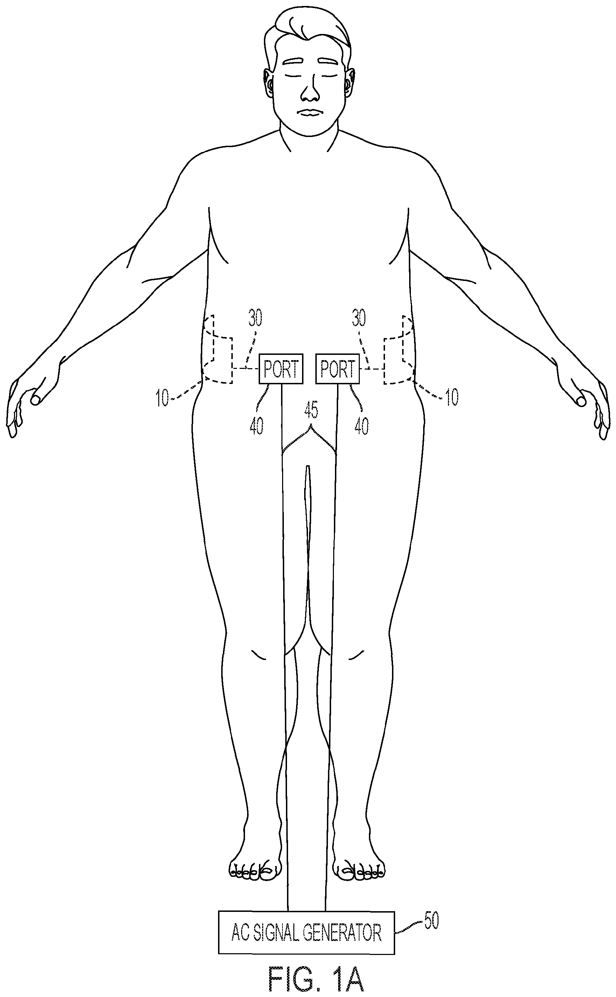

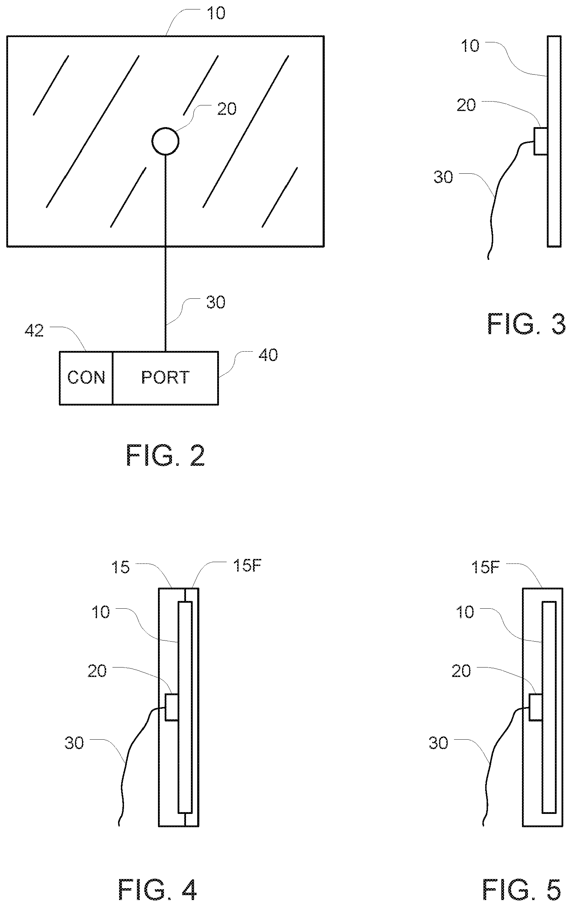

FIG. 1A depicts two sheets of graphite 10 that are implanted in a subject's body on opposite sides of a target region located within the subject's body. Each sheet of graphite has a corresponding port 40, and a wire 30 or another electrical conductor routes an electrical signal between each port 40 and the respective sheet of graphite 10. The ports 40 are designed to facilitate safe attachment and detachment of the distal end of a cable 45 from outside the body. This may be accomplished, for example, by incorporating a connector into each of the ports 40, as described below in connection with FIG. 2. The proximal ends of both cables 45 terminate at an AC signal generator 50. As a result, when the AC signal generator 50 imposes an AC signal between the two cables 45, a corresponding voltage will appear between the two implanted sheets of graphite 10.

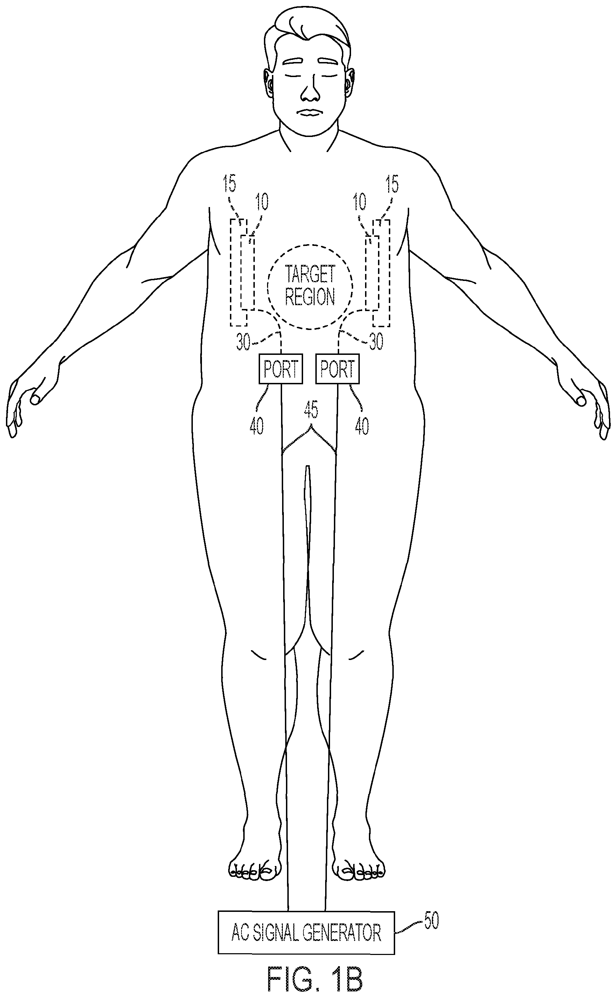

FIG. 1A shows the graphite sheets implanted subcutaneously adjacent to the subject's skin. However, in other embodiments, the graphite sheets may be implanted further into the subject's body. For example, the graphite sheets may be located adjacent to, and on either side of, a target (such as a tumor) in a target region or in a target location, or may be adjacent to, or wrapped around, an organ of the body within which the target is located (e.g., as depicted in FIG. 1B). The graphite sheets may be supported on a substrate or by a backing layer. FIG. 1B depicts graphite sheets implanted further into the subject's body and incorporating a sheet of biocompatible material 15 positioned behind the rear face of the sheet of graphite 10, and this sheet 15 is configured to support the rear face of the sheet of graphite 10. Suitable materials for the sheet 15 include, for example, biocompatible polymers, ceramics, composites, metals, etc. (e.g., acrylic polymers, titanium metal). Alternatively, the sheet of biocompatible material 15 may be a sheet of graphite mesh.

Optionally, additional sheets of graphite (not shown) may be implanted in the subject's body. For example, in addition to the pair of implanted sheets of graphite 10 on the right and left sides of the subject's abdomen illustrated in FIG. 1, an additional pair of graphite sheets may be implanted on the front and back of the subject's abdomen. When additional sheets of graphite are provided, signals from the AC signal generator 50 are provided to those graphite sheets at appropriate times. Further optionally, a third pair of graphite sheets may be implanted in like manner. Again, additional implanted graphite sheets may be implanted subcutaneously adjacent to the subject's skin, or may be implanted further into the subject's body.

In further embodiments, one or more electrodes may be implanted within the body, and one or more electrodes may be positioned outside the body. For example, one (or more) pair of electrodes may be implanted in the body, while one (or more) pair of electrodes may be positioned outside the body. Alternatively, one electrode of a pair of electrodes may be implanted in the body, while the other of that pair of electrodes may be positioned outside the body; etc. It is to be understood that, throughout this disclosure, these options are included even though not specifically recited. That is, reference to two electrodes or two sheets of graphite implanted in the subject's body or positioned subcutaneously within the subject's body refers also to at least one electrode or sheet of graphite implanted in the subject's body or positioned subcutaneously within the subject's body and one or more electrodes or sheets of graphite positioned externally on the subject's body or clothing. In each case, the sheets of graphite are positioned so that the front face of each sheet of graphite faces the target region in the subject's body.

The location at which the sheets of graphite 10 are installed will depend on the position of the tumor that is being treated. Examples of appropriate locations include subcutaneous locations just below the surface of the subject's skin, between the subject's skin and skull, or just beneath the subject's skull. Other appropriate locations include positioning one or more sheets of graphite 10 deeper within a subject's brain (e.g., in a resection cavity that has been formed during surgery), or positioning one or more sheets of graphite within the subject's torso, such as within the abdomen (e.g., on either side of an organ such as the pancreas).

The sheet of graphite 10 could be made of pyrolytic graphite, graphitized polymer film, graphite foil made from compressed high purity exfoliated mineral graphite, or other forms of graphite. The sheet of graphite has anisotropic properties with respect to both electrical conductivity properties and thermal conductivity properties, such that the sheet spreads out both the heat and the current more evenly over a larger surface area.

The anisotropic thermal properties include directional thermal properties. Specifically, the sheet has a first thermal conductivity in a direction that is perpendicular to its front face. And the thermal conductivity of the sheet in directions parallel to the front face is more than two times higher than the first thermal conductivity. In some preferred embodiments, the thermal conductivity in the parallel directions is more than ten times higher than the first thermal conductivity. For example, the thermal conductivity of the sheet in directions that are parallel to the front face may be more than: 1.5 times, 2 times, 3 times, 5 times, 10 times, 20 times, 100 times, 200 times, or even more than 1,000 times higher than the first thermal conductivity.

The anisotropic electrical properties include directional electrical properties. Specifically, the sheet has a first resistance in a direction that is perpendicular to its front face. And the resistance of the sheet in directions parallel to the front face is less than the first resistance. In some preferred embodiments, the resistance in the parallel directions is less than half of the first resistance or less than 10% of the first resistance. For example, the resistance of the sheet 10 in directions that are parallel to the front face may be less than: 75%, 50%, 40%, 30%, 20%, 10%, 5%, 1%, 0.5%, or even less than 0.1% of the first resistance.

For example, the thermal conductivity of a sheet of pyrolytic graphite in directions that are in the x-y plane is between 10 times and 20 times higher than its thermal conductivity in the perpendicular z-direction; and the electrical resistivity of a sheet of pyrolytic graphite in directions that are in the x-y plane is approximately three orders of magnitude (1,000 times) lower than its electrical resistivity in the perpendicular z-direction.

Examples of suitable forms of graphite include synthetic graphite, such as pyrolytic graphite (including, but not limited to, Pyrolytic Graphite Sheet (PGS), available from Panasonic Industry, Kadoma, Osaka, Japan), or graphitized polymer film, e.g., graphitized polyimide film, (including, but not limited to, that supplied by Kaneka Corp., Moka, Tochigi, Japan. Graphite foil made from compressed high purity exfoliated mineral graphite may also be suitable (for example, but not limited to, that supplied by MinGraph^{®} 2010A Flexible Graphite, available from Mineral Seal Corp., Tucson, Arizona, USA). In alternative embodiments, conductive anisotropic materials other than graphite may be used instead of graphite.

FIG. 2 depicts an apparatus for applying an alternating electric field to a target region in a subject's body. This apparatus includes a sheet of graphite 10 that has a front face and a rear face. The sheet of graphite 10 could be, for example, a non-porous, continuous sheet with no openings or pores that pass therethrough, or a continuous sheet with openings or pores that pass therethrough. The sheet of graphite 10 may be flexible to facilitate implantation, and may be implanted into the subject's body using techniques that are similar to the techniques for implanting hernia meshes into a subject's body, including but not limited to laparoscopic techniques. When flexible sheets of graphite are used, they can be inserted into the subject's body through a small incision in a folded or rolled-up state. The sheet of graphite 10 is then subsequently unfolded or unrolled at the desired location within the subject's body to make a larger surface area available. The flexibility of the graphite sheet helps to minimize the size of the surgical incision and procedure.

A port 40 is configured for affixation to a living subject's body. The port(s) may be located directly adjacent to the implanted sheets of graphite, in near proximity to the implanted sheets of graphite, or may traverse the skin at a location some distance from the implanted sheets of graphite. The port has an outer surface that includes or connects to a mating electrical connector 42. The affixation of the port 40 to the subject's body may be implemented by implanting the port such that the connecter 42 remains accessible from the outside. The connector 42 is configured to accept the cable 45 (shown in FIGS. 1A and 1B). Preferably, the connector 42 is configured so that the cable 45 can be disconnected, e.g., when the subject does not want to be encumbered by those cables.

One or more electrical conductors are positioned to route an electrical signal between the electrical connector 42 and the sheet of graphite. In the embodiment illustrated in FIG. 2, these conductors are implemented using a terminal 20 (e.g., a conductive metal) that is attached to the sheet of graphite 10, a wire 30, and portions of the port 40. A side view of portions of this embodiment are shown in FIG. 3. The wire 30 in these embodiments could be, for example, a biocompatible metal wire or a graphite wire. And the terminal 20 in these embodiments could be connected to the sheet of graphite 10 using, for example, a conductive adhesive. In alternative embodiments, the wire 30 can be omitted, in which case the port 40 would be connected directly to the sheet of graphite 10 using, for example, a suitable conductive adhesive. In these latter examples, the conductive portions of the port 40 and the conductive adhesive serve as the electrical conductors.

Referring now to FIGS. 1A-3, the sheets of graphite 10 are positioned subcutaneously or implanted within the subject's body, with the front face of the sheet of graphite facing the target region. The mating electrical connector 42 is positioned to be accessible from outside the subject's body. When the cables 45 are attached to the ports 40 via the electrical connector 42, the respective sheet of graphite 10 can be energized by the AC signal generator 50 via the respective cables 45, ports 40, and wire/conductor 30. When an AC voltage is applied between the two sheets of graphite 10, an electric field will be conductively coupled into the region that lies between those two sheets of graphite (which includes the target region in the subject's body).

In some embodiments, a layer of a biocompatible insulating polymer material 15F may be disposed on the front face of the sheet of graphite 10, as shown in FIG. 4. The insulating polymer material has a dielectric constant of at least 10 (or, in some embodiments, at least 20).

Suitable materials for use as the insulating polymer material 15F include poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF - TrFE - CtFE)" and "Poly(VDF - TrFE - CFE)", respectively. Optionally, ceramic nanoparticles may be mixed into the Poly(VDF - TrFE - CtFE) and/or Poly(VDF - TrFE - CFE) to form a "nanocomposite." Optionally, these ceramic nanoparticles may comprise ferroelectric metal oxides (e.g., barium titanate and/or barium strontium titanate). In alternative embodiments, a different polymer that provides a high dielectric constant (e.g., at least 10 or at least 20) may be used. Examples of alternative polymeric materials that may be used in place of Poly(VDF - TrFE - CtFE) and/or Poly(VDF - TrFE - CFE) include the following: (1) ceramic nanoparticles mixed into at least one of Poly(VDF - TrFE), P(VDF - HFP), PVDF, or other polymers (where HFP is hexafluoropropylene); and (2) barium titanate and/or barium strontium titanate ceramic nanoparticles mixed into at least one of Poly(VDF - TrFE), P(VDF - HFP), PVDF. In other embodiments, the insulating polymer material 15F is formed by mixing ceramic nanoparticles into at least one other polymer.

In some embodiments, including the embodiment depicted in FIG. 4, a sheet of biocompatible material 15 is positioned behind the rear face of the sheet of graphite 10, and this sheet 15 is configured to support the rear face of the sheet of graphite 10. Suitable materials for the sheet 15 include, for example, biocompatible polymers, ceramics, composites, metals, etc. (e.g., acrylic polymers, titanium metal). Alternatively, the sheet of biocompatible material 15 may be a sheet of graphite mesh. Optionally, the sheet of biocompatible material may also be included in other embodiments herein, such as, for example, the embodiment shown in FIG. 3, configured to support the rear face of the sheet of graphite.

In some embodiments, the biocompatible insulating polymer material 15F is disposed on both faces of the sheet of graphite 10, as shown in FIG. 5. The insulating polymer material has a dielectric constant of at least 10 (or, in some embodiments, at least 20), and may be made from the same materials as the layer 15F described above in connection with FIG. 4.

To use the embodiments described above in connection with FIG. 4-5, two sheets of graphite 10 are positioned subcutaneously or implanted within the subject's body, with the front face of each sheet of graphite facing the target region. When an AC voltage is applied between the two sheets of graphite 10, an electric field will be capacitively coupled (due to the polymer layer 15F) into the region that lies between those two sheets of graphite (which includes the target region in the subject's body).

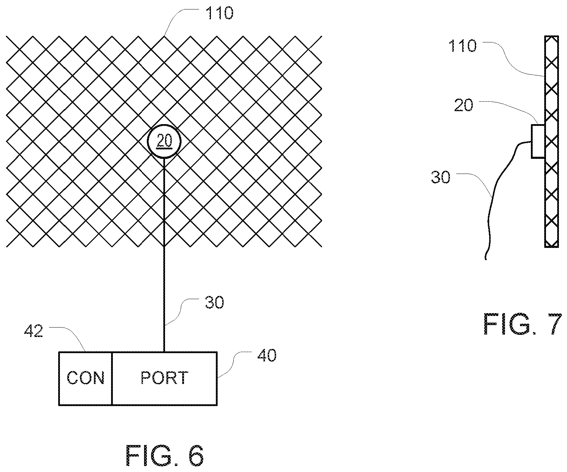

FIGS. 6 and 7 are plan and side views of another apparatus for applying an alternating electric field to a target region in a subject's body. This apparatus is similar to the embodiment described above in connection with FIGS. 2 and 3, except that in place of the sheets of graphite described above in connection with FIGS. 2 and 3, the sheet of graphite is a sheet-shaped piece of graphite mesh 110. The sheet of graphite mesh 110 may be flexible to facilitate implantation, and may be implanted into the subject's body using techniques that are similar to the techniques for implanting hernia meshes into a subject's body (e.g., as described above in connection with FIG. 2). The sheet of graphite mesh 110 could be made of pyrolytic graphite, graphitized polymer film, graphite foil made from compressed high purity exfoliated mineral graphite, or other forms of graphite.

To use the FIG. 6-7 embodiments, two sheets of graphite mesh 110 are positioned subcutaneously or implanted within the subject's body, with the front face of each sheet of graphite facing the target region. When an AC voltage is applied between the two sheets of graphite mesh 110, an electric field will be conductively coupled into the region that lies between those two sheets of graphite mesh (which includes the target region in the subject's body). Variations of the embodiments of FIGS. 4 and 5 may be applicable for the embodiments of FIGS. 6 and 7.

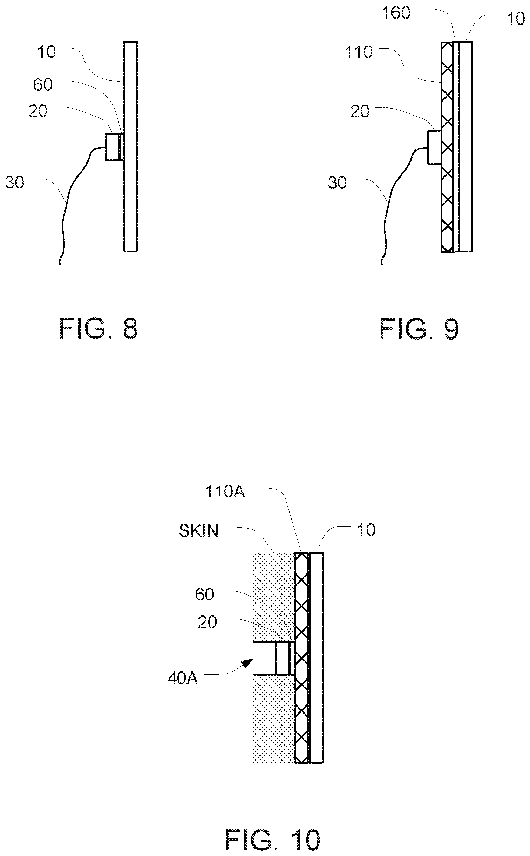

FIG. 8 is a side view of another apparatus for applying an alternating electric field to a target region in a subject's body. This apparatus is similar to the embodiment described above in connection with FIGS. 2 and 3, except that an additional layer of material 60 with a high dielectric constant (e.g., at least 10 or at least 20) is positioned between the terminal 20 and the sheet of graphite 10. When this embodiment is used for implantation into a subject's body, and an AC voltage is applied between two sheets of graphite 10 implanted in the body, an electric field will be capacitively coupled into the region that lies between those two sheets of graphite 10, which includes the target region in the subject's body. The layer of material 60 may be formed from high dielectric constant ceramic materials or from the same high dielectric constant polymeric materials as the layer 15F described above in connection with FIG. 4. Keeping the layer of material 60 with the high dielectric constant thin (e.g., less than 20 µm, less than 10 µm, or less than 5 µm) is preferable in order to improve the capacitive coupling. Variations of the embodiments of FIGS. 4 and 5 or FIGS. 6 and 7 may be applicable for the FIG. 8 embodiment.

FIG. 9 is a side view of another apparatus for applying an alternating electric field to a target region in a subject's body. This apparatus is similar to the embodiment described above in connection with FIGS. 2 and 3, except that two additional layers are included. More specifically, a layer of graphite mesh 110 is positioned behind the sheet of graphite 10, and an additional layer of material 160 with a high dielectric constant (e.g., at least 10 or at least 20) is positioned between the layer of graphite mesh 110 and the sheet of graphite 10. When this embodiment is positioned subcutaneously or implanted within the subject's body, and an AC voltage is applied between two sheets of graphite 10 implanted in the body, an electric field will be capacitively coupled into the region that lies between those two sheets of graphite 10, which includes the target region in the subject's body. The layer of material 160 may be formed from high dielectric constant ceramic materials or from the same high dielectric constant polymeric materials as the layer 15F described above in connection with FIG. 4, and the layer 160 is preferably thin (e.g., less than 20 µm, less than 10 µm, or less than 5 µm).

In other embodiments, layers 110 and 160 in FIG. 9 may be reversed and the resulting apparatus may operate similarly. In other embodiments, the layer of graphite mesh 110 may be replaced with a second sheet of graphite 10 such that the layer of material 160 with a high dielectric constant (e.g., at least 10 or at least 20) is positioned between two sheets of graphite 10, and the resulting apparatus may operate similarly.

FIG. 10 is a side view of another apparatus for applying an alternating electric field to a target region in a subject's body. This apparatus is similar to the embodiment described above in connection with FIGS. 2 and 3, except that instead of connecting the sheet of graphite 10 to the port (e.g., port 40 in FIGS. 1A, 1B, 2, and 6) via a wire 30, the sheet of graphite 10 and a port 40A are combined into an integrated unit that is implanted into the subject's body just beneath the subject's skin. The electrical connection to the sheet of graphite 10 is made via this port 40A.

In the embodiment illustrated in FIG. 10, a support layer made from graphite mesh 110A is positioned behind the sheet of graphite 10. But in alternative embodiments, this support layer could be made from a different material (e.g., an acrylic polymer) or omitted entirely.

In the embodiment illustrated in FIG. 10, a layer of material 60 with a high dielectric constant (e.g., at least 10 or at least 20) is interposed between the electrical terminal 20 and the sheet of graphite 10. When this layer of material 60 is included, and an AC voltage is applied between two implanted sheets of graphite 10 (e.g., via two ports 40A), an electric field will be capacitively coupled into the region that lies between those two sheets of graphite 10, which includes the target region in the subject's body. The layer of material 60 may be formed from high dielectric constant ceramic materials or from the same high dielectric constant polymeric materials as the layer 15F described above in connection with FIG. 4, and the layer 60 is preferably thin (e.g., less than 20 µm, less than 10 µm, or less than 5 µm).

In alternative embodiments, the layer of material 60 can be omitted, in which case the terminal 20 of the port 40A makes electrical contact with the sheet of graphite 10, either directly, or via optional support layer 110A. In these alternative embodiments, when an AC voltage is applied between the two sheets of graphite 10 (e.g., via two ports 40A), an electric field will be conductively coupled into the region that lies between those two sheets of graphite 10, which includes the target region in the subject's body. Optionally, electrical contact may be enhanced between terminal 20 of the port 40A and the sheet of graphite 10, or the support layer 110A, by using a connecting layer of a conductive adhesive.

FIG. 10 illustrates one electrode assembly situated directly adjacent to the port 40A. However, other layered constructs of the electrode assembly described herein may also be used in this manner and, indeed, situating the electrode assembly directly adjacent to the port 40A may be preferable for the embodiments incorporating the high dielectric material layer (e.g., 15F in FIG. 4 and 5; 60 in FIG. 8; and 160 in FIG. 9).

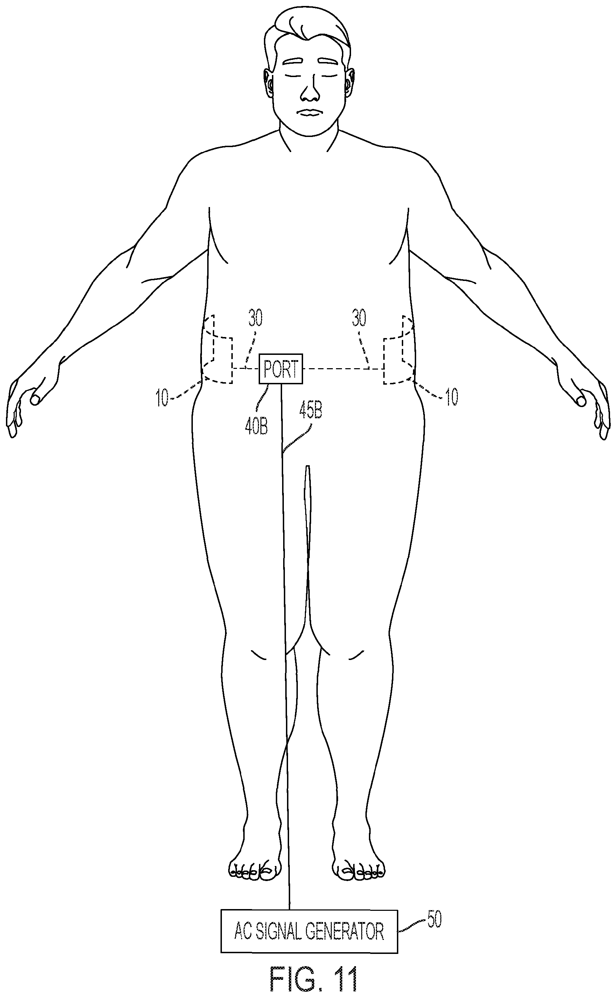

FIG. 11 depicts another approach for connecting the implanted graphite sheets that is similar to the approach depicted in the embodiments of FIGS. 1A and 1B, except that instead of providing a separate port 40 for connecting with each sheet of graphite, respectively, this FIG. 11 approach utilizes a single multi-conductor port 40B that accepts a multi-conductor cable 45B from the AC signal generator 50. An individual wire 30 or other electrical conductor runs through the subject's body to route one of the electrical signals that arrives via the multi-conductor cable 45B between the single port 40B and one sheet of graphite 10. And a second wire 30 (or other electrical conductor) runs through the subject's body to route the other electrical signal that arrives via the multi-conductor cable 45B between the single port 40B and the other sheet of graphite 10. In this FIG. 11 embodiment, the construction of the sheet of graphite 10 and the connections thereto (e.g., using the wires 30) may be as described above in connection with FIGS. 2-9. When more than two sheets of graphite are implanted into the subject's body, the single port 45B can direct signals to all of the implanted sheets 10 (e.g., using an individual wire 30 for each implanted sheet of graphite).

The hardware described above in connection with FIG. 1-11 may be used to practice the following method of applying an alternating electric field to a target region in a subject's body. The method comprises applying an alternating voltage between a first sheet of graphite 10 that has been previously implanted in the subject's body and a second sheet of graphite 10 that has been previously implanted in the subject's body, wherein the first sheet of graphite and the second sheet of graphite are positioned on opposite sides of the target region. In some embodiments, the frequency of the alternating voltage is between 50 kHz and 1 MHz, or between 100 kHz and 500 kHz.

At some point in time prior to the applying, the first and second sheets of graphite 10 are implanted in the subject's body.

Optionally, a layer of a biocompatible insulating polymer material 15F having a dielectric constant of at least 10 is disposed on at least one face of the first sheet of graphite 10, and a layer of a biocompatible insulating polymer material 15F having a dielectric constant of at least 10 is disposed on at least one face of the second sheet of graphite 10.

Optionally, the first sheet of graphite 10 is supported by a first sheet of biocompatible material 15, and the second sheet of graphite is supported by a second sheet of biocompatible material 15. In each case, the biocompatible support may be a sheet of graphite mesh.

The sheets of graphite 10 may be made of a synthetic graphite. The sheets of graphite 10 may be made of pyrolytic graphite, graphitized polymer film, or graphite foil made from compressed high purity exfoliated mineral graphite. In some embodiments, the sheets of graphite 10 may be oxidized (or partially oxidized) sheets of graphite, or the sheets may be constructed of oxidized (or partially oxidized) graphite, which may be used to improve adhesion to the sheets of graphite. As described above in connection with FIG. 6-7, the first sheet of graphite could be a mesh sheet 110.

In any of the embodiments and methods described herein, sensors for measuring temperature (such as thermistors) may be positioned on or near the graphite sheets 10 so that tissue temperature can be controlled and thermal damage to tissue avoided.

In any of the embodiments and methods described herein, adhesion between components or layers of the electrode assembly, or between the electrode assembly and the surrounding tissues, can be achieved using a conductive adhesive.

In any of the embodiments and methods described herein, it is preferred to connect internal wiring/circuitry to external wiring/circuitry outside of the body via one or more ports positioned on or across the subject's skin, as indicated in FIGS. 1A, 1B and 11. However, in alternative embodiments, the one or more ports are not present and a separate connection is utilized outside the body (for example, one or more connectors). The use of a port has the advantage of allowing the subject to disconnect the external wiring at a connector at the port, thereby allowing the subject to be unencumbered of external wiring at chosen times.

In any of the embodiments and methods described herein, it is preferred to locate the electric field generator and/or AC signal generator outside of the body, as indicated in FIGS. 1A, 1B and 11, which also show a wired connection with components inside the body. However, in alternative embodiments, the external device may communicate wirelessly with components that are inside the body. It is desirable to minimize the number of transcutaneous wires. For example, a small electronics package may be implanted in the body, connected by a single wire (for example, the same wire that supplies the electric field) to the external unit and electronics. The implanted electronics package may include circuitry and switches that can be controlled wirelessly from outside the body.

The implanted electrodes of the embodiments described herein advantageously may be significantly smaller than prior art implantable electrodes. In some embodiments, the surface area of the sheets of graphite (and also the front face of the electrode assembly) may be from approximately 1-40 cm² or greater, and the electrode assembly may have a thickness of less than 1 mm, or may be greater than 1 mm.

Optionally, in addition to implanting two sheets of graphite on opposite sides of a target region as described above, and subsequently applying an AC voltage between those two sheets of graphite in order to induce an alternating electric field in the target region, an additional two sheets of graphite may be implanted in the subject's body on opposite sides of the target region. For example, if the original two sheets of graphite are implanted on the left and right sides of a target region, the additional two sheets of graphite should be implanted in front of and in back of the target region. When an additional two sheets of graphite are implanted, applying an AC voltage between the original two sheets of graphite will impose an alternating electric field with a first orientation in the target region, and applying an AC voltage between the additional two sheets of graphite will impose an alternating electric field with a second orientation in the target region. Details of construction of the additional sheets of graphite, as well as structures for supporting those sheets, are as described above in connection with FIGS. 2-10.

In alternative embodiments, instead of implanting two sheets of graphite on opposite sides of a target region, and applying an AC voltage between those two sheets of graphite in order to induce an alternating electric field in the target region (as described above), only a single sheet of graphite can be implanted on one side of the target region. In these embodiments, a second electrode (which may or may not be constructed using graphite) is positioned outside the subject's body on the opposite side of the target region, and an AC voltage is applied between the implanted sheet of graphite and the second electrode (i.e., the external electrode).

In these embodiments, an alternating electric field is applied to a target region in a subject's body by applying an alternating voltage between a sheet of graphite that has been previously implanted in the subject's body and a second electrode that is positioned outside the subject's body. Details of the construction of the implanted sheet of graphite (as well as structures for supporting the sheet of graphite) are as described above in connection with FIGS. 2-10. The sheet of graphite and the second electrode are positioned on opposite sides of the target region. Prior to applying the alternating voltage, the first sheet of graphite is implanted in the subject's body, and the second electrode is positioned in contact with the subject's body.

Embodiments illustrated under any heading or in any portion of the disclosure may be combined with embodiments illustrated under the same or any other heading or other portion of the disclosure unless otherwise indicated herein or otherwise clearly contradicted by context.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. An apparatus for applying an alternating electric field to a target region in a subject's body, the apparatus comprising:
first and second sheets of graphite (10), wherein the sheets of graphite (10) each have a front face and a rear face and are configured separately to be implantable or positionable subcutaneously in the subject's body, with the front faces of the sheets of graphite (10) facing the target region;
one or more ports (40; 40B) configured for affixation to the subject's body,
wherein the one or more ports (40; 40B) have an outer surface which includes an electrical connector (42) accessible from outside of the subject's body; and
an AC signal generator (50) configured to impose an alternating voltage, at a frequency between 50 kHz and 1 MHz, between the sheets of graphite (10).

2. The apparatus of claim 1, wherein one or each of the sheets of graphite (10) is a mesh sheet (110).

3. The apparatus of claim 1, wherein one or each of the sheets of graphite (10) incorporates a layer of biocompatible material (15) configured to support the rear face thereof, optionally the layer of biocompatible material (15) is a sheet of graphite mesh.

4. The apparatus of claim 1, wherein one or each of the sheets of graphite (10) incorporates a layer of biocompatible insulating polymer material (15, 15F) disposed on at least one of the faces thereof, and the insulating polymer material has a dielectric constant of at least 10, optionally one or each of the sheets of graphite (10) incorporates a layer of biocompatible insulating polymer material (15, 15F) disposed on the front and rear faces thereof.

5. The apparatus of claim 1, wherein one or each of the sheets of graphite (10) incorporates a layer of biocompatible insulating polymer material (15F) disposed on the front face thereof, and the insulating polymer material has a dielectric constant of at least 10.

6. The apparatus of claim 1, wherein one or each of the sheets of graphite (10) comprises pyrolytic graphite, graphitized polymer film or graphite foil made from compressed, high-purity exfoliated mineral graphite, or at least partially oxidized forms thereof.

7. The apparatus of claim 1, wherein one or each of the sheets of graphite (10) is a non-porous, continuous sheet with no openings.

8. The apparatus of claim 1, wherein one or each of the sheets of graphite (10) is a continuous sheet with openings or pores passing therethrough.

9. The apparatus of claim 1, wherein one or each of the sheets of graphite (10) is a flexible sheet, and one or each of the sheets of graphite (10) is folded or rolled-up and configured to be unfolded or unrolled when implanted or positioned in the subject's body.

10. The transducer apparatus of claim 1, further comprising:
electrical conductors (30) positioned to route an electrical signal between the one or more ports (40; 40B) and the sheets of graphite (10).

11. The transducer apparatus of claim 10, wherein the electrical conductors (30) comprise a biocompatible electrically-conductive wire, optionally a metal wire or a graphite wire.

12. The transducer apparatus of claim 1, wherein the one or more ports are first and second ports (40), wherein the first and second ports (40) are connected to respective ones of the sheets of graphite (10), optionally the first and second ports (40) are connected directly to respective ones of the sheets of graphite (10).

13. The transducer apparatus of claim 1, wherein the one or more ports (40; 40B) include an electrical connector (42) configured to accept electrical cables (45), optionally the electrical connector (42) is configured so that the electrical cables (45) can be detached.

14. The transducer apparatus of claim 1, wherein the one or more ports is a single port (40B) which is connected to each of the sheets of graphite (10).

15. The transducer apparatus of claim 1, wherein one or more of the sheets of graphite (10) incorporate a layer of graphite mesh (110) behind the rear face thereof and an additional layer of material (160) positioned between the sheet of graphite (10) and the layer of graphite mesh (110), and the additional layer of material (160) has a dielectric constant of at least 10, optionally the additional layer of material (160) has a thickness of less than 20 µm.

## Patentansprüche

1. Einrichtung zum Anlegen eines elektrischen Wechselfeldes an einen Zielbereich im Körper eines Probanden, wobei die Einrichtung Folgendes umfasst:
erste und zweite Graphitfolien (10), wobei die Graphitfolien (10) jeweils eine Vorder- und eine Rückseite aufweisen und separat so konfiguriert sind, dass sie subkutan im Körper des Probanden implantiert oder positioniert werden können, wobei die Vorderseiten der Graphitfolien (10) der Zielregion zugewandt sind;
einen oder mehrere Anschlüsse (40; 40B) zur Befestigung am Körper des Probanden, wobei der eine oder die mehreren Anschlüsse (40; 40B) eine Außenfläche aufweisen, die einen von außen zugänglichen elektrischen Anschluss (42) umfasst; und
einen Wechselstromsignalgenerator (50), der so konfiguriert ist, dass er eine Wechselspannung mit einer Frequenz zwischen 50 kHz und 1 MHz zwischen die Graphitfolien (10) anlegt.

2. Einrichtung nach Anspruch 1, wobei eine oder jede der Graphitfolien (10) eine Gewebefolie (110) ist.

3. Einrichtung nach Anspruch 1, wobei eine oder jede der Graphitfolien (10) eine Schicht aus biokompatiblem Material (15) einbezieht, die so konfiguriert ist, dass sie die Rückseite davon trägt, wobei die Schicht aus biokompatiblem Material (15) optional eine Folie aus Graphitgewebe ist.

4. Einrichtung nach Anspruch 1, wobei eine oder jede der Graphitfolien (10) eine Schicht aus biokompatiblem isolierendem Polymermaterial (15, 15F) einbezieht, die auf mindestens einer ihrer Seiten angeordnet ist, und das isolierende Polymermaterial eine Dielektrizitätskonstante von mindestens 10 aufweist, wobei optional eine oder jede der Graphitfolien (10) eine Schicht aus biokompatiblem isolierendem Polymermaterial (15, 15F) einbezieht, die auf der Vorder- und Rückseite davon angeordnet ist.

5. Einrichtung nach Anspruch 1, wobei eine oder jede der Graphitfolien (10) eine Schicht aus biokompatiblem isolierendem Polymermaterial (15F) auf der Vorderseite davon einbezieht und das isolierende Polymermaterial eine Dielektrizitätskonstante von mindestens 10 aufweist.

6. Einrichtung nach Anspruch 1, wobei eine oder jede der Graphitfolien (10) pyrolytischen Graphit, graphitierten Polymerfilm oder Graphitfolie aus komprimiertem, hochreinem, exfoliertem Mineralgraphit oder mindestens teilweise oxidierte Formen davon umfasst.

7. Einrichtung nach Anspruch 1, wobei eine oder jede der Graphitfolien (10) eine nicht poröse, durchgehende Schicht ohne Öffnungen ist.

8. Einrichtung nach Anspruch 1, wobei eine oder jede der Graphitfolien (10) eine durchgehende Folie mit Öffnungen oder Poren ist, die durch diese hindurch verlaufen.

9. Einrichtung nach Anspruch 1, wobei eine oder jede der Graphitfolien (10) eine flexible Folie ist und eine oder jede der Graphitfolien (10) gefaltet oder aufgerollt ist und so konfiguriert ist, dass sie sich beim Einsetzen oder Positionieren im Körper des Probanden entfalten oder abrollen kann.

10. Wandlereinrichtung nach Anspruch 1, weiter umfassend:
elektrische Leiter (30), die so positioniert sind, dass sie ein elektrisches Signal zwischen dem einem oder den mehreren Anschlüssen (40; 40B) und den Graphitfolien (10) leiten.

11. Wandlereinrichtung nach Anspruch 10, wobei die elektrischen Leiter (30) einen biokompatiblen, elektrisch leitfähigen Draht, optional einen Metalldraht oder einen Graphitdraht, umfassen.

12. Wandlereinrichtung nach Anspruch 1, wobei es sich bei dem einen oder den mehreren Anschlüssen um einen ersten und einen zweiten Anschluss (40) handelt, wobei der erste und der zweite Anschluss (40) mit jeweils einem der Graphitfolien (10) verbunden sind, optional wobei der erste und der zweite Anschluss (40) direkt mit jeweils einer der Graphitfolien (10) verbunden sind.

13. Wandlereinrichtung nach Anspruch 1, wobei der eine oder die mehreren Anschlüsse (40; 40B) einen elektrischen Anschluss (42) einschließen, der zur Aufnahme elektrischer Kabel (45) konfiguriert ist; optional, wobei der elektrische Anschluss (42) so konfiguriert ist, dass die elektrischen Kabel (45) abgenommen werden können.

14. Wandlereinrichtung nach Anspruch 1, wobei es sich bei dem einen oder den mehreren Anschlüssen um einen einzigen Anschluss (40B) handelt, der mit jeder der Graphitfolien (10) verbunden ist.

15. Wandlereinrichtung nach Anspruch 1, wobei eine oder mehrere der Graphitfolien (10) eine Schicht aus Graphitgewebe (110) hinter ihrer Rückseite einbezieht und eine zusätzliche Materialschicht (160) zwischen der Graphitfolie (10) und der Schicht aus Graphitgewebe (110) positioniert ist, und die zusätzliche Materialschicht (160) eine Dielektrizitätskonstante von mindestens 10 aufweist, wobei die zusätzliche Materialschicht (160) optional eine Dicke von weniger als 20 µm aufweist.

## Revendications

1. Appareil permettant d'appliquer un champ électrique alternatif à une région cible du corps d'un sujet, cet appareil comprenant :
une première et une seconde feuilles de graphite (10), dans lequel les feuilles de graphite (10) présentent chacune une face avant et une face arrière et sont configurées séparément pour être implantables ou positionnables par voie sous-cutanée dans le corps du sujet, les faces avant des feuilles de graphite (10) faisant face à la région cible ;
un ou plusieurs ports (40 ; 40B) configurés pour être fixés au corps du sujet, dans lequel le ou les ports (40 ; 40B) présentent une surface extérieure qui inclut un connecteur électrique (42) accessible de l'extérieur du corps du sujet ; et
un générateur de signal CA (50) configuré pour imposer une tension alternative, à une fréquence comprise entre 50 kHz et 1 MHz, entre les feuilles de graphite (10).

2. Appareil selon la revendication 1, dans lequel une ou chacune des feuilles de graphite (10) est une feuille de maille (110).

3. Appareil selon la revendication 1, dans lequel une ou chacune des feuilles de graphite (10) incorpore une couche de matériau biocompatible (15) configurée pour supporter sa face arrière, la couche de matériau biocompatible (15) étant optionnellement une feuille de maille de graphite.

4. Appareil selon la revendication 1, dans lequel une ou chacune des feuilles de graphite (10) incorpore une couche de matériau polymère isolant biocompatible (15, 15F) disposée sur au moins une de ses faces, et le matériau polymère isolant présente une constante diélectrique d'au moins 10, optionnellement une ou chacune des feuilles de graphite (10) incorpore une couche de matériau polymère isolant biocompatible (15, 15F) disposée sur ses faces avant et arrière.

5. Appareil selon la revendication 1, dans lequel une ou chacune des feuilles de graphite (10) incorpore une couche de matériau polymère isolant biocompatible (15F) disposée sur sa face avant, et le matériau polymère isolant présente une constante diélectrique d'au moins 10.

6. Appareil selon la revendication 1, dans lequel une ou chacune des feuilles de graphite (10) comprend du graphite pyrolytique, un film polymère graphitisé ou une feuille de graphite fabriquée à partir de graphite minéral exfolié compressé de haute pureté, ou au moins des formes partiellement oxydées de celui-ci.

7. Appareil selon la revendication 1, dans lequel une ou chacune des feuilles de graphite (10) est une feuille continue non poreuse sans ouvertures.

8. Appareil selon la revendication 1, dans lequel une ou chacune des feuilles de graphite (10) est une feuille continue avec des ouvertures ou des pores la traversant.

9. Appareil selon la revendication 1, dans lequel une ou chacune des feuilles de graphite (10) est une feuille flexible, et une ou chacune des feuilles de graphite (10) est pliée ou enroulée et configurée pour être dépliée ou déroulée lorsqu'elle est implantée ou positionnée dans le corps du sujet.

10. Appareil transducteur selon la revendication 1, comprenant en outre :
des conducteurs électriques (30) positionnés pour acheminer un signal électrique entre le ou les ports (40 ; 40B) et les feuilles de graphite (10).

11. Appareil transducteur selon la revendication 10, dans lequel les conducteurs électriques (30) comprennent un fil électriquement conducteur biocompatible, éventuellement un fil métallique ou un fil de graphite.

12. Appareil transducteur selon la revendication 1, dans lequel le ou les ports sont un premier et un second ports (40), dans lequel le premier et le second ports (40) sont connectés à des feuilles de graphite respectives (10), en option le premier et le second ports (40) sont connectés directement à des feuilles de graphite respectives (10).

13. Appareil transducteur selon la revendication 1, dans lequel le ou les ports (40 ; 40B) incluent un connecteur électrique (42) configuré pour accepter des câbles électriques (45), le connecteur électrique (42) étant éventuellement configuré de manière à ce que les câbles électriques (45) puissent être détachés.

14. Appareil transducteur selon la revendication 1, dans lequel le ou les ports sont un seul port (40B) qui est connecté à chacune des feuilles de graphite (10).

15. Appareil transducteur selon la revendication 1, dans lequel une ou plusieurs des feuilles de graphite (10) incorporent une couche de maille de graphite (110) derrière leur face arrière et une couche supplémentaire de matériau (160) positionnée entre la feuille de graphite (10) et la couche de maille de graphite (110), et la couche supplémentaire de matériau (160) présente une constante diélectrique d'au moins 10, optionnellement la couche supplémentaire de matériau (160) présente une épaisseur inférieure à 20 µm.
